# EUROPÄISCHE PATENTSCHRIFT

(11) **EP 0 600 317 B1**
(45) Veröffentlichungstag und Bekanntmachung des Hinweises auf die Patenterteilung: **12.03.1997**
(21) Anmeldenummer: 93118601.9
(22) Anmeldetag: 18.11.1993
(51) Int. Cl.: C07C 63/70, C07C 51/285, C07C 51/34, C07C 37/60, C07C 39/27

(54) **Verfahren zur Herstellung von halogenierten Benzoesäuren**
Process for the preparation of halogenated benzoic acids
Procédé pour la préparation d'acides benzoiques halogénés

(30) Priorität: 29.11.1992 DE 4240020
(43) Veröffentlichungstag der Anmeldung: 08.06.1994
(73) Patentinhaber: HOECHST AKTIENGESELLSCHAFT, 65926 Frankfurt am Main (DE)
(72) Erfinder: Pfirmann, Ralf, Dr., D-64347 Griesheim (DE)

(56) Entgegenhaltungen:
- EP-A- 0 564 333
- JOURNAL OF THE AMERICAN CHEMICAL SOCIETY, Bd. 72, Nr. 12, 2. Januar 1951, Washington, DC, US, Seiten 5515-5518, W. VON DOERING et al: "The peracetic acid cleavage of unsymmetrical ketones"

## Beschreibung

Die vorliegende Erfindung betrifft ein neues, vorteilhaftes Verfahren zur Herstellung von halogenierten Benzoesäuren und gegebenenfalls von halogenierten Phenolen aus halogenierten, insbesondere fluorierten Benzophenonen, gegebenenfalls in einem Verfahrensschritt. Die als Ausgangsverbindungen eingesetzten halogenierten Benzophenone sind teilweise bekannt, teilweise neu. Die neuen Ausgangsverbindungen und ihre Herstellung sind in der Patentanmeldung P 42 40 022.8 vom gleichen Anmeldedatum beschrieben. Die erfindungsgemäß herstellbaren halogenierten Benzoesäuren und halogenierten Phenole stellen Zwischenprodukte dar, und können nach literaturbekannten Verfahren in Pharmazeutika, Pflanzenschutzmittel und flüssigkristalline Verbindungen umgewandelt werden. Das erfindungsgemäße Verfahren hat gegenüber bekannten Verfahren ökonomische und ökologische Vorteile, da nur wenige, relativ einfache Umsetzungsstufen und günstige, zum Teil auch recyclisierbare Reagenzien eingesetzt werden.

Es ist bekannt, daß halogenierte Benzoesäuren, wie beispielsweise die 2,4,5-Trifluorbenzoesäure, geeignete Vorprodukte für antibakteriell wirksame Chinoloncarbonsäurederivate sind (DE-A 3 318 145; EOS 340055; JP 01/128978). Das erfindungsgemäße Verfahren stellt eine ökonomisch günstige Alternative zu den bekannten Verfahren zur Herstellung von beispielsweise 2,4-Dichlor-5-fluorbenzoesäure, 2-Chlor-4,5-difluorbenzoesäure, 2-Chlor-4-fluorbenzoesäure und 2,3,4,5-Tetrafluorbenzoesäure sowie 2,4-Dichlorphenol, 2,6-Difluorphenol, 2-Fluorphenol und 4-Fluorphenol dar. 2,4-Dichlor-5-fluorbenzoesäure kann nach literaturbekannten Methoden (DE-OS 3 702 393; DE-OS 3 615 767; DE-OS 3 601 567, DE-OS 3 517 535, DE-OS 3 142 854) in antibakteriell wirksame Fluorchinoloncarbonsäuren umgewandelt werden. Für 2-Chlor-4,5-difluorbenzoesäure sind derartige Umsetzungen in EOS 342 849 und EOS 321 191 beschrieben. 2,3,4,5-Tetrafluorbenzoesäure wird nach EOS 153 163, DE-OS 3 517 535, DE-OS 3 409 922, DE-OS 3 318 145 zu hochwirksamen antibakteriellen Mitteln umgesetzt. 2,6-Difluorphenol kann zu festen, leitfähigen Polymeren (JP 01/170 010) zum Bau von Kondensatoren umgesetzt werden, ebenso wie zu Monomeren (EOS 308 863), zu Herbiziden (EOS 142 328, US 4 750 931), Insektiziden und Acariziden (GB 2 187 452, GB 2 199 825) und antiviral aktiven Verbindungen (J. Med. Chem. 32 (2) [1989], 450-455, G. D. Diana). Aus 4-Fluorphenol lassen sich nach literaturbekannten Methoden unter anderem Antiarrhythmika (EOS 454 498), Phospholipase-Inhibitoren (US 5 070 207), Fungizide und psychoaktive Substanzen (EOS 449 187; EOS 428 437), Mittel gegen Arthritis und Osteoporose (EOS 403 185, A. H. Robins Co.), Diuretika, Antihypertensiva, Antikoagulantien und Lipoxygenaseinhibitoren (FR 2 653 119) und flüssigkristalline Verbindungen (EOS 442 306) herstellen. 2-Fluorphenol kann nach beschriebenen Methoden zum Beispiel zu speziellen Farbstoffen (JP 03/255 086), zu ferroelektrischen Flüssigkristallen (JP 03/197 438), zu psychotropen Verbindungen (EOS 428 437), zu Entkrampfungsmitteln (US 5 025 031) und zu auf das Zentralnervensystem wirksamen Substanzen (EOS 417 027) umgesetzt werden. Aus 2-Chlor-4-fluorphenol lassen sich zum Beispiel vorteilhafte Herbizide (JP 02/048 571; EOS 271 170) und Fungizide (EOS 326 330) herstellen. 3-Chlor-4-fluorphenol dient zum Beispiel zur Herstellung von Mitteln zur Behandlung von Diabetes (EOS 230 379) und Vorprodukten für Flüssigkristalle (JP 59/210 048; JP 59/175 453). Für 2-Chlor-4-fluorbenzoesäure sind Methoden bekannt, welche die Umsetzung zu Cholesterol-Biosynthese-Hemmern (EOS 431 480) und Herbiziden (Synthesis (1987), 883-887, T.N. Wheeler et al.; PCT WO 8 707 602) erlauben. Aus 2,4-Dichlorphenol lassen sich zum Beispiel Anthelmintika und Antiseptika (FR 2 658 192) Herbizide (JP 03/193 771), Polymere mit Absorptionseigenschaften für ultraviolettes Licht (JP 03/200 788) und molekulare Sonden zur Detektion der Hydrolyseeigenschaften von Enzymen (JP 03/215 462) herstellen.

Die Vorteile des erfindungsgemäßen Verfahrens gegenüber den bisher bekannten Verfahren zur Herstellung der wertvollen halogenierten Benzoesäuren sollen am Beispiel der 2-Chlor-4,5-difluorbenzosäure, erläutert werden.

Durch Chlor-Fluor-Austausch (Halex-)-Reaktion von 2,4-Dichlor-5-fluorbenzonitril (EOS 431 373) im technisch unerwünschten Lösungsmittel Dimethylsulfoxid unter Verwendung von sprühgetrocknetem Kaliumfluorid erhält man 2-Chlor-4,5-difluorbenzonitril, das nach literaturbekannten Methoden (Houben-Weyl-Müller, Methoden der Organischen Chemie, Bd. VIII (1952), S. 429-433) in die Benzoesäure umgewandelt werden kann. In diesem Falle, auch bei Verwendung für die Umsetzung weniger aktiver Lösungsmittel wie zum Beispiel Sulfolan (EOS 433 124), erhält man 2,4,5-Trifluorbenzonitril als Nebenprodukt in Ausbeuten um 20 %, wodurch erhöhter Fluoridverbrauch und Zersetzungsanteil erhalten werden. Ungünstiger noch ist aber der in diesem Fall im 2-Chlor-4,5-difluorbenzonitril enthaltene Anteil an 4-Chlor-2,5-difluorbenzonitril, der je nach Umsatz zwischen etwa 3 % und etwa 8 % liegt und in aufwendiger Weise abgetrennt werden muß. Das 2,4-Dichlor-5-fluorbenzonitril muß selbst aufwendig (EOS 433 124) durch Bromierung von 2,4-Dichlorfluorbenzol und anschließenden Brom-Cyan-Austausch mittels Alkalicyaniden hergestellt werden.

Ebenso ist selektiver Chlor-Fluor-Austausch des 4-Chlor-Atoms in 2,4-Dichlor-5-fluorbenzoesäureestern bekannt (EOS 355 774), jedoch ist die Herstellung der Ausgangsmaterialien recht ungünstig, insbesondere deshalb, als lediglich höhere und verzweigte Ester bei der Austauschreaktion befriedigende Ausbeuten ergeben. Bei einfacher zu erhaltenden niederen Alkylestern werden aufgrund starker Zersetzung bei Reaktionstemperatur schlechte Ausbeuten erhalten, weil die Estergruppierung lediglich geringe Aktivierung einbringt, gegen Nucleophile jedoch reaktiv ist.

Durch Fluorierung von 4,5-Dichlorphtalsäureanhydrid (DE-OS 3 303 378, EOS 55 630, EOS 55 629, JP 02/76 872) erhält man 4,5-Difluorphthalsäureanhydrid, das durch Umsetzung mit Ammoniak und Hofmann-Abbau in 4,5-Difluoranthranilsäure (EOS 55 630, US 4 994 606) und durch chlorierende Sandmeyer-Reaktion (JP 02/215 744) in 2-Chlor-4,5-difluorbenzoesäure überführt werden kann. Durch Decarboxylierung der 4,5-Difluorphthalsäure kann man nach verschiedenen Methoden (EOS 429 848, JP 01/25 737, JP 63/295 529) 3,4-Difluorbenzoesäure erhalten, die durch Nitrierung und denitrierende Chlorierung (US 5 003 103) in das Zielprodukt umgewandelt werden kann. Diese alternativen Synthesen sind zum Teil aufgrund ihrer Vielstufigkeit und schlechten Gesamtausbeuten, zum Teil durch Werkstoffprobleme und reaktionstechnische Schwierigkeiten ungünstig.

Wirtschaftlich ungünstig sind allgemein Acylierungen von 1,2,4-Trifluorbenzol und 3,4-Difluorchlorbenzol mittels unter Umständen aliphatisch chlorierten Acetylchloriden und anschließender Haloform-Reaktion mittels Hypchlorit-Lösungen (EOS 411 252, DE 3 840 371, DE 3 840 375, EOS 303 291), da 1,2,4-Trifluorbenzol und 3,4-Dichlorfluorbenzol (über 1,2-Difluorbenzol durch Chlorierung oder ausgehend von 2,5-Dichlornitrobenzol [T.F. Braish et al., Org. Prep. Proced. Int. 23 (1991), 655-658] oder denitrierende Chlorierung von 3,4-Difluornitrobenzol) selbst durch sehr aufwendige Schritte wie Balz-Schiemann-Reaktion unter hohen Ausbeuteverlusten hergestellt werden müssen und die Abbaureaktionen hohe Salzbelastungen im Abwasser erzeugen.

Die erfindungsgemäße Umsetzung basiert auf einem literaturbekannten Reaktionstyp, der Baeyer-Villiger-Oxidation (Baeyer, Villiger, Ber. 32 (1899), 3625). Bei dieser Reaktion wird ein Keton, seltener ein Aldehyd, mit Persäuren unter verschiedenen Reaktionsbedingungen (Baeyer, loc. cit.; W. Dilthey, Ber. (1939), 219-237; Wacek, Bezard, Ber. 74 (941), 845; Wittig, Pieper, Ber. 73 (1940), 295) umgesetzt. Makroskopisch beobachtet man eine Umsetzung zu Estern, was mikroskopisch durch eine anionotrope Wanderung eines Restes der Carbonylgruppe erklärt wird. Bekannt ist, daß Methylgruppen bzw. Wasserstoffatome sehr geringe Wanderungstendenzen besitzen, was auch technische Anwendung findet. Die Umsetzung einfacher, unsymmetrischer Ketone, insbesondere von Benzophenonen wurde bereits von W. v. E. Doering et al. (J. Am. Chem. Soc. (1950), 5515-5518) untersucht, jedoch waren die Selektivitäten ebenso wie die Ausbeuten und Umsätze zum Teil gering, da es in der Absicht der Autoren lag, mechanistische Aspekte zu untersuchen. Insbesondere die Umsetzung mehrfach halogenierter Benzophenone war noch nicht untersucht worden. Einen älteren Hinweis auf die Selektivität solcher Reaktionen in Anwesenheit von Nitrogruppen findet man in Ber. 71 (1938) (W. Dilthey et al.). Aus 4-Nitrobenzophenon erhält man nach dieser Untersuchung 4-Nitrobenzoesäure und Phenol.

Gegenstand der Erfindung ist ein neues, vorteilhaftes Verfahren zur Herstellung von halogenierten Benzoesäuren der allgemeinen Formel (1) 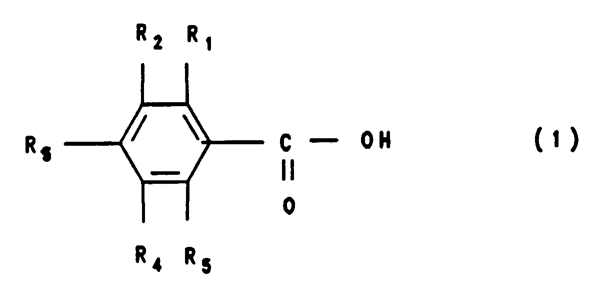 in welcher R₁, R₂, R₃, R₄ und R₅ Wasserstoff-, Fluor-, Chlor- oder Bromatome, Alkyl (C₁-C₆)-, Alkoxy(C₁-C₆)-, Nitro-, Cyano-, Trifluormethyl-, Aldehyd-, Alkoxy(C₁-C₄)-carbonyl-, -SO₂-Alkyl(C₁-C₄)-, SO₂-Phenyl-, -CONH₂-, -CON(Alkyl(C₁-C₄))₂-, Hydroxy-, -Carboxyl-, -NH₂- oder -N(Alkyl-(C₁-C₄))₂-gruppen bedeuten, wobei mindestens einer der Substituenten R₁ - R₅ eines der genannten Halogenatome ist, dadurch gekennzeichnet, das man 1 Mol eines hinsichtlich der Benzolkerne A und B asymmetrisch substituierten Benzophenons der allgemeinen Formel (2) 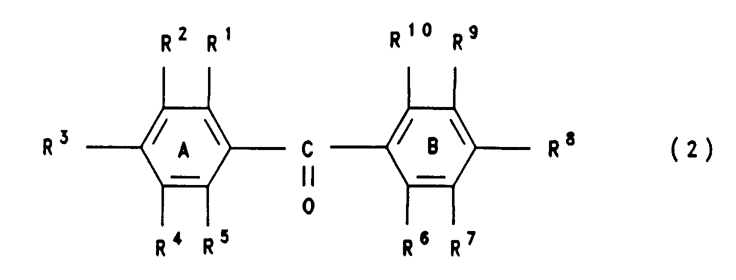 in welcher R¹ - R¹⁰ die vorstehend für R₁ - R₅ genannte Bedeutungen haben, mit 1 bis 10 Mol, vorzugsweise 1,05 bis 3 Mol, eines Oxidationsmittels aus der Reihe Wasserstoffperoxid, Harnstoff-Wasserstoffperoxid-Additionsprodukt, Alkalimetallperoxide, wie Natriumperoxid, Ammonium-, Alkalimetall- oder Erdalkalimetallperoxodisulfate, -perwolframate, -perborate, -percarbonate, Ozon, Alkyl- oder Arylpercarbonsäuren, Alkyl- oder Arylpersulfonsäuren oder Perschwefelsäure (Caro'sche Säure), ggfs. in Gegenwart von gegenüber den Reaktionsteilnehmern inerten anorganischen und/oder organischen Lösungsmitteln bei Temperaturen von -20 ° bis + 100 °C, vorzugsweise von 0° bis 70 °C, besonders bevorzugt von 20 bis 60 °C bei Atmosphärendruck, Überdruck oder Unterdruck umsetzt.

An geeigneten Oxidationsmitteln seien im einzelnen noch folgende genannt:

Natrium-, Kalium- oder Ammoniumperoxodisulfat, Natriumpercarbonat, Perameisensäure, Peressigsäure, Trifluorperessigsäure, Hexafluorpropanpersulfonsäure, 3-Chlorperbenzoesäure, 3,5-Dinitroperbenzoesäure, Magnesiummono-perphthalat, Methanpersulfonsäure, Trifluormethanpersulfonsäure oder p-Toluolpersulfonsäure oder ® Oxone (= Kaliumperoxomonosulfat).

Es ist vielfach zweckmäßig, die erfindungsgemäße Umsetzung in gegenüber den Reaktionsteilnehmern inerten anorganischen oder organischen Lösungsmitteln vorzunehmen. Solche Lösungsmittel sind beispielsweise Schwefelsäuren verschiedener Konzentration, beispielsweise solche von 80 Gewichtsprozent bis 20 gewichtsprozentigem Oleum, vorzugweise 95 gewichtsprozentiger Schwefelsäure bis 5 gewichtsprozentigem Oleum, ferner Fluorwasserstoff, aliphatische Carbonsäuren von 1 bis 6 Kohlenstoffatomen, die im Alkylrest durch Halogenatome substituiert sein können, Alkylsulfonsäuren von 1 bis 6 Kohlenstoffatomen, die im Alkyrest durch Halogenatome substituiert sein können, oder Acetanhydrid.

Anstelle eines der vorstehend genannten Lösungsmittel oder zusätzlich, das heißt in Kombination, kann ein inertes organisches Lösungsmittel, wie beispielsweise Dichlormethan, Trichlormethan, 1,2-Dichlorethan, Toluol, ein Xylol, Chlorbenzol, Dichlorbenzol, Chlortoluol oder Dichlortoluol angewandt werden.

Bei der einfachsten, bevorzugten Durchführungsform des erfindungsgemäßen Verfahrens wird in Schwefelsäure verschiedener Konzentration unter Zudosierung von Wasserstoffperoxid gearbeitet. Dabei wird in der Regel Schwefelsäure bzw. Oleum der weiter oben genannten Konzentrationen verwendet. Die umzusetzenden Benzophenone der genannten allgemeinen Formel (2) können in diesem Medium problemlos gelöst werden, wobei zwischen 200 Massenprozent und 2000 Massenprozent Schwefelsäure eingesetzt werden. Dieser Mischung wird Wasserstoffperoxid-Lösung zudosiert. Man verwendet hierbei Lösungen eines Gehalts von 10 bis 90 Gewichtsprozent, bevorzugt handelsübliche Lösungen mit Gehalten von 25 bis 50 Gewichtsprozent. Diese niedrig konzentrierten Lösungen haben Vorteile bei der Handhabung und Sicherheit. Verdünntere Lösungen ergeben zu große Wärmetönungen beim Zudosieren aufgrund der Hydratationswärme der Schwefelsäure. Es versteht sich von selbst, daß vorgemischte Schwefelsäure-Wasserstoffperoxid-Lösungen dieselben Ergebnisse ergeben. Die Umsetzung wird bei den weiter oben genannten Temperaturen durchgeführt. Die Reaktionszeiten liegen zwischen 1 h und 16 h, in den meisten Fällen zwischen 3 und 8 h, je nach umzusetzendem Benzophenon. Die Dosierzeiten können der Wärmetönung entsprechend gewählt werden.

Nach beendeter Umsetzung können die erhaltenen Benzoesäuren durch Verdünnen der Reaktionsmischung und Filtration isoliert werden, gegebenenfalls ist eine Extraktion der in Wasser gelösten Substanz notwendig. Die gegebenenfalls parallel enstandenen Phenole der allgemeinen Formel (3) 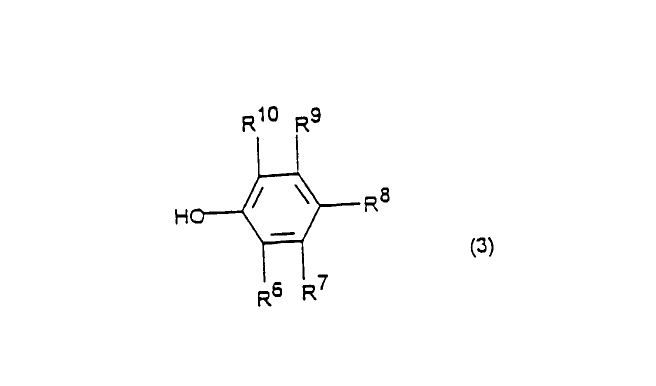 in welcher R₆-R₁₀ die gennanten Bedeutungen haben, wobei mindestens einer der Substituenten R₆-R₁₀ ein Fluoratom darstellt, lassen sich im allgemeinen am günstigsten nach dem Verdünnen der Reaktionsmischung (Schwefelsäure-Konzentration zwischen 50 und 80 Gewichtsprozent) und gegebenenfalls mehrstündigem Erhitzen zur Desulfonierung (Gilbert, Sulfonation and Related Reactions, S. 427-442) durch Wasserdampfdestillation isolieren. Extraktive Abtrennung aus der Reaktionsmischung und anschließende destillative Trennung der Reaktionsprodukte gelingt in den meisten Fällen ebenfalls problemlos.

Im Zuge der Aufarbeitung von fluorhaltigen Verbindungen kann es zur Verhinderung von Fluorwasserstoffkorrosion sinnvoll sein, in Gegenwart von fluoridfangenden Mitteln wie Calciumsalzen oder Siliciumdioxid zu arbeiten. Geeignete Calciumsalze bzw. -verbindungen sind beispielsweise Calciumchlorid, -sulfat oder -hydroxid.

Setzt man erfindungsgemäß unsymmetrische Benzophenone der genannten allgemeinen Formel (2) ein, so erhält man unter den Bedingungen der beschriebenen bevorzugten Ausführungsform, ohne in einer Phase der Umsetzung substituierte Benzoesäurephenylester nachweisen zu können, substituierte Benzoesäuren und Phenole. Man erhält überraschenderweise sehr selektiv nur eine Kombination dieser Produkte, weil die unterschiedlichen Arylreste unter den erfindungsgemäßen Bedingungen deutlich unterschiedliche Wanderungstendenz besitzen. Es entsteht ein Phenol aus dem Rest des Benzophenons, der weniger elektronenanziehende Substituenten enthält. Dabei kann die Regel gelten, daß elektronenspendende Gruppen, wie zum Beispiel Alkyl(C₁-C₄)-, Alkoxy(C₁-C₄)- oder Arylgruppen in ihrer Wirkung im Sinne der Erfindung durch dieselbe Anzahl elektronenanziehender Gruppen, wie Halogenatome oder Nitrogruppen, ausgeglichen werden. Analoges kann für den anderen Arylrest gelten, aus dem wegen der geringeren Wanderungstendenz die entsprechende halogenierte Benzoesäure entsteht. Diese Selektivität wird beim erfindungsgemäßen Verfahren beobachtet und ist Gegenstand der Erfindung, wenn die Zahl der elektronenanziehenden Reste im Ring A um mindestens 1 höher ist als die Zahl der elektronenanziehenden Reste, vermindert um die Zahl der Alkyl- oder Alkoxygruppen, im Ring B.

Die nachstehende Beispiele dienen zur Erläuterung des erfindungsgemäßen Verfahrens, ohne es darauf zu beschränken.

### Beispiel 1

26,8 g (0,1 Mol) 2,4-Dichlor-5-fluorbenzophenon werden in 250 g 96 %iger Schwefelsäure gelöst und unter guter Durchmischung bei 0°C mit 17 g 30 %iger Wasserperoxid-Lösung so versetzt, daß die Temperatur nicht über 40 °C steigt. Nach der Zudosierung läßt man auf 20 °C kommen und rührt 5 h bei dieser Temperatur. Bei unvollständiger Umsetzung dosiert man 5 g Wasserstoffperoxidlösung nach (T < 40 °C) und verdünnt nach 3 h weiterer Reaktionszeit mit 1000 g Wasser und rührt 2 h nach. Die ausgefallene 2,4-Dichlor-5-fluorbenzoesäure wird abfiltriert und durch Umkristallisation (Wasser) gereinigt. Man erhält 17,8 g (0,0852 Mol, 85 %) 2,4-Dichlor-5-fluorbenzoesäure vom Schmelzpunkt 144-146 °C.

### Beispiel 2

Einer Lösung von 37,8 g (0,15 Mol) 2-Chlor-4,5-difluorbenzophenon in 400 g 100 %iger Schwefelsäure tropft man binnen 30 min 32 g 20 %iger Wasserstoffperoxidlösung so zu, daß die Temperatur unterhalb 35 °C bleibt.

Nach 7 h ist die Umsetzung vollständig. Anschließend wird die Reaktionsmischung auf 1200 g Eis gegeben. Durch Extraktion der Mutterlauge mit Methyl-tert.butylether, Isolierung des Rohproduktes aus dem Extrakt und Kristallisation aus Wasser erhält man 17,0 g (0,0883 Mol, 88 %) farblose 2-Chlor-4,5-difluorbenzoesäure vom Schmelzpunkt 104,4 - 105,8 °C.

### Beispiel 3

28,7 g (0,1 Mol) 2,4-Dichlor-4',5-difluorbenzophenon werden in 250 g 10 %igem Oleum gelöst und bei Temperaturen bis 30 °C mit 25 g 50%iger Wasserstoffperoxidlösung versetzt. Man erwärmt nach Dosierende (30 min) 8 h auf 50 °C und gibt nach Kühlung auf 250 g Eis. Diese Mischung wird 8 h bei 140 °C gehalten und 4-Fluorphenol anschließend durch Wasserdampfdestillation gewonnen (7,5 g, 0,0673 Mol, 67 %, Fp. (DSC) 42,6 °C). 2,4-Dichlor-5-fluorbenzoesäure wird, wie in Beispiel 1 beschrieben, isoliert (17,0 g, 0.0813 Mol, 81 %, Fp. 143-145°C).
(DSC = differential scanning Calorimetry)

### Beispiel 4

Zu 61,0 g (0,25 Mol) 2,4-Dichlor-2'(4')-methylbenzophenon in 300 g 90 %iger Schwefelsäure werden 11 g 90 % Wasserstoffperoxid-Lösung bei 0-10 °C zugetropft. Man rührt 4 h nach und gibt auf 800 g Eis. Durch Filtration isoliert man 39,2 g (0,206 Mol, 83 %) beigefarbene 2,4-Dichlorbenzoesäure (Schmelzpunkt 151-157 °C). Durch Erhitzen (6 h) der wäßrigen Phase auf 120 °C und Wasserdampfdestillation kann eine Mischung von o- und p-Kresol isoliert werden (24,5 g).

### Beispiel 5

Setzt man 59,0 g (0,25 Mol) 2,4,5-Trifluorbenzophenon mit 53,3 g 20 prozentiger Wasserstoffperoxid-Lösung ein und verfährt im übrigen wie in Beispiel 2 angegeben, so erhält man 34,7 g (0, 197 Mol, 79 %) 2,4,5-Trifluorbenzoesäure vom Schmelzpunkt 99,6-101,6 °C.

### Beispiel 6

Setzt man 60,5 g (0,2 Mol) 2,4,5-Trichlor-2'-fluorbenzophenon und 50 g 50 prozentige Wasserstoffperoxid-Lösung ein und führt die Umsetzung im übrigen wie in Beispiel 3 beschrieben aus, so isoliert man durch Filtration nach Verdünnen und Ausrühren der Mutterlauge (2 h) 40,8 g (0,18 Mol, 90 %) 2,4,5-Trichlorbenzoesäure (roh, RG (GC) ca. 85 %, Schmelzpunkt 158-165°C) und 12,3 g (0,11 Mol, 55 %) 2-Fluorphenol (RG (GC) > 97 %).

### Beispiel 7

Zu 500 g 96 %iger Schwefelsäure wurden 33,7 g (0,1 Mol) 2,3,4,5-Tetrachlor-2'(4')-fluorbenzophenon gegeben und die dunkelbraune Lösung bei 20 °C mit 25 g (0,22 Mol) 30 %iger Wasserstoffperoxid-Lösung versetzt. Dabei wurde die Temperatur bei 55 °C konstant gehalten. Nach weiteren 9 h bei 60 °C wurde auf 500 g Eis gegeben und 6 h zu Rückfluß erhitzt. Durch anschließende Wasserdampfdestillation erhält man ein Gemisch aus 0,7 g (0,006 Mol, 6 %) 2-Fluorphenol und 6,4 g (0,57 Mol, 57 %) 4-Fluorphenol. Die Phenole lassen sich bei Ansatzvergrößerung durch Fraktionierung trennen, wobei bei 150-155 °C 2-Fluorphenol und bei 177-182 °C (101,3 KPa) 4-Fluorphenol übergeht. Nach Abkühlen erhält man durch Filtrieren der Mutterlauge 2,3,4-5-Tetrachlorbenzoesäure als hellbraunen Festkörper (24.1 g roh, Schmelzpunkt 186-192 °C).

### Beispiel 8

Durch Umsetzung von 37,1 g (0,1 Mol) 2,2',3,5,6-Pentachlor-4'-fluorbenzophenon in 350 g 100 %iger Schwefelsäure mit 20 g (0,176 Mol) 30 %iger Wasserstoffperoxidlösung bei 60 °C (8 h) und anschließendes Verdünnen mit Wasser (500 g) wurde 2,3,5,6-Tetrachlorbenzoesäure in Suspension erhalten und abfiltriert (23,2 g, 0,089 Mol, 89 %, Schmelzpunkt 171-178 °C (roh)). Durch Erhitzen des Filtrats auf 150 °C (4 h) und Wasserdampfdestillation in Anwesenheit von 10 g Calciumchlorid isoliert man 10,3 g (0,070 Mol, 70 %) farbloses 2-Chlor-4-fluorphenol (Ep. 21,4 °C).

### Beispiel 9

Arbeitet man wie in Beispiel 8 beschrieben, setzt jedoch 30,7 g (0,1 Mol) 3'-Chlor-2,3,4,4',5-pentafluorbenzophenon ein, so erhält man nach Wasserdampfdestillation 8,5 g (0,058 Mol, 58 %) 3-Chlor-4-fluorphenol und durch Extraktion mit Methyl-tert.butylether und Einengen des Extraktes aus der Mutterlauge 15,4 g (0,079 Mol, 79 %) hellbraune 2,3,4,5-Tetrafluorbenzoesäure. Diese kann durch Umkristallisation aus Wasser gereinigt werden (Fp. 85,5-86 °C).

### Beispiel 10

Arbeitet man unter Einsatz von 35,6 g (0,1 Mol) 2,3,4,5-Tetrachlor-2'-6'-difluorbenzophenon wie in Beispiel 8 (Reaktionstemperatur 70 °C) beschrieben, so erhält man 3,1 g (0,024 Mol, 24 %) 2,6-Difluorphenol und 22,4 g (0,086 Mol, 86 %) 2,3,4,5-Tetrachlorbenzoesäure (roh, RG (GC) ca. 95 %).

### Beispiel 11

Aus 45,8 g (0,15 Mol) 2(5),2'-Dichlor-3,4,5(2)-trifluorbenzophenon erhält man unter den Bedingungen des Beispiels 8 mittels 30 g 30 prozentiger Wasserstoffperoxid-Lösung ein Isomerengemisch aus 2-Chlor-3,4-5-trifluorbenzoesäure und 5-Chlor-2,3,4-trifluorbenzoesäure im Verhältnis 1:1,5 (24,1 g, 0,114 Mol, 76 %) und nach Wasserdampfdestillation 8,7 g (0,068 Mol, 45 %) 2-Chlorphenol.

### Beispiel 12

Durch Umsetzung von 35,8 g (0,1 Mol) 2-Brom-4-chlor-5-fluor-3'-nitrobenzophenon in 150 g 5%igem Oleum mit 9,7 g (0,2 Mol) 70 %iger Wasserstoffperoxidlösung erhält man nach Verdünnen (300 g Wasser), 4 h Erhitzen zum Rückfluß und Extraktion mit Methyl-tert.butylether nach Entfernen des Lösungsmittels ein Gemisch (29,0 g) aus 2-Brom-4-chlor-5-fluorphenol, 2-Brom-4-chlor-5-fluorbenzoesäure, 3-Nitrophenol und 3-Nitrobenzoesäure (Anteile GC-Flächen-%: 14, 35, 36, 15).

### Beispiel 13

30,8 g (0,1 Mol) 2-Brom-4-fluor-3',5'-dimethylbenzophenon wurden in 140 g 90 %iger Schwefelsäure gelöst und zwischen -10 °C und 0 °C 5,7 g 90 %iger Wasserstoffperoxidlösung zugetropft (Dosierzeit 1 h). Nach einer Nachrührzeit von 2 h wurde auf 500 g Eis gegeben und anschließend 5 h zum Sieden erhitzt. Durch Isolierung, wie in Beispiel 8 beschrieben, wurden 9,6 g (0,079 Mol, 79 %) 3,5-Dimethylphenol und 15,6 g (0,071 Mol, 71 %) 2-Brom-4-fluorbenzoesäure (roh, RG (GC) ca. 90 %, Schmelzpunkt 160-166 °C) erhalten.

### Beispiel 14

Arbeitet man unter Einsatz von 27,6 g (0,1 Mol) 2,4-Difluor-3',5'-dimethoxybenzophenon, jedoch sonst wie in Beispiel 13 beschrieben, so erhält man 12,4 g (0,081 Mol, 81 %) 3,5-Dimethoxyphenol und 13,2 g (0,084 Mol, 84 %) 2,4-Difluorbenzoesäure (Fp. 183-185 °C).

### Beispiel 15

Arbeitet man wie in Beispiel 13 beschrieben, jedoch unter Verwendung von 50,1 g (0,175 Mol) 2,3-Dichlor-4,4'-difluorbenzophenon und 10 g 90 prozentiger Wasserstoffperoxid-Lösung, so ergeben sich 11,8 g (0,105 Mol, 60 %) 4-Fluorphenol und 28,3 g (0,136 Mol, 78 %) 2,3-Dichlor-4-fluorbenzoesäure.

### Beispiel 16

30,9 g (0,125 Mol) 2-Chlor-4-fluor-4'-methylbenzophenon werden, wie in Beispiel 13 beschrieben, mit 7,1 g 90 prozentiger Wasserstoffperoxid-Lösung in 19,6 g (0,112 Mol, 90 %) 2-Chlor-4-fluorbenzoesäure und 10,6 g (0,098 Mol, 78 %) p-Kresol überführt.

### Beispiel 17

23,5 g (0,1 Mol) 3,4-Difluor-4'-methylbenzophenon können, wie in Beispiel 13 beschrieben, in 11,9 g (0,075 Mol, 75 %) 3,4-Difluorbenzoesäure und 9,5 g (0,088 Mol, 88 %) p-Kresol überführt werden.

### Beispiel 18

28,1 g (0,1 Mol) 2,5-Dichlor-4'-methoxybenzophenon werden, wie in Beispiel 13 beschrieben, in 15,8 g (0,083 Mol, 83 %) 2,5-Dichlorbenzoesäure (Schmelzpunkt 150-155,5 °C) und 5,0 g (0,04 Mol, 40 %) Hydrochinonmonomethylether überführt.

### Beispiel 19

Zu 5,4 g (20 mMol) 2,4-Dichlor-5-fluorbenzophenon (roh, 11 % Isomerengehalt) in 40 g 1,2-Dichlorethan wurden unter Rühren bei 20 - 25 °C 7,9 g 30 %ige Wasserstoffperoxid-Lösung und 20 g Ameisensäure zugetropft. Danach erhitzte man 20 h zum Sieden, wobei nach 10 h erneut Wasserstoffperoxid-Ameisensäure-Mischung zugegeben wurde. Danach wurde mit 100 g Wasser verdünnt, die Phasen getrennt und die organische Phase über Magnesiumsulfat getrocknet. Nach Filtration und Entfernen des Lösungsmittels wurde 5 h mit 50 g 50 %iger Schwefelsäure auf 120 °C erhitzt (Spaltung teilweise enthaltenen 2,4-Dichlor-5-fluorbenzoesäurephenylesters) und dann mit 100 g Wasser verdünnt. Die ausgefallene 2,4-Dichlor-5-fluorbenzoesäure (2,8 g (roh), 13,4 Mol, 68 % bez. Reingehalt (GC) kann zur Reinigung aus Wasser umkristallisiert werden.

### Beispiel 20

In 5 g (19,8 mMol) 2-Chlor-4,5-difluorbenzophenon und 20 g Eisessig trägt man 8 g Ammoniumperoxodisulfat ein und erhitzt auf 90 °C (12 h). Man gibt anschließend 100 g 70 %ige Schwefelsäure zu und erhitzt 2 h auf 150 °C. Durch Verdünnen mit 200 g Wasser und Filtration erhält man 3,1 g (16,0 mMol, 81 %) 2-Chlor-4,5-difluorbenzoesäure (RG (GC) ca. 92 %). Durch Umlösung aus Wasser erhält man 2,5 g Produkt vom Schmelzpunkt 104 °C - 105 °C.

### Bespiel 21

Zu einer Mischung aus 2,0 g (8 mMol) 2-Chlor-4-fluor-4'-methylbenzophenon, 9,9 g (0,07 Mol) Dinatriumhydrogenphosphat und 3,8 g (40 mMol) Harnstoff-Wasserstoffperoxid-Addukt in 50 ml Dichlormethan wurden bei 0°C 2,1 g (20 mMol) Acetanhydrid zugetropft. Dann ließ man auf Raumtemperatur erwärmen. Nach 12 h bei 20 °C wurde mit Natriumhydrogencarbonat-Lösung neutralisiert und die wäßrige Phase mit Dichlormethan nachextrahiert. Anschließend wurde das Lösungsmittel aus der organischen Phase im Vakuum abdestilliert. Zum Rückstand (2,5 g, enthaltend ca. 80 % 2-Chlor-4-fluorbenzoesäurephenylester) wurden 50 g 70 %ige Schwefelsäure gegeben und 6 h auf Siedetemperatur erhitzt. Durch Verdünnen der erkalteten Mischung und Extraktion mit Methyl-tert.-butylether, Trocknen, Entfernen des Lösungsmittels und Umlösung des Rohproduktes aus Wasser erhält man 1,0 g (5,7 mMol, 72 %) 2-Chlor-4-fluorbenzoesäure in Form eines farblosen Pulvers (Fp. 182-186 °C).

### Beispiel 22

Zu 3,8 g (12 mMol) 3-Chlorperbenzoesäure (55 %ig) in 40 ml Dichlormethan und 14,2 g (0,1 Mol) Dinatriumhydrogenphosphat tropfte man unter Inertgas bei 20 °C 2,3 g (10 mMol) 2-Methyl-4-fluor-4'-methylbenzophenon und erwärmte dann 8 h auf 40 °C. Die Reaktionsmischung wurde gaschromatographisch überwacht und bei unvollständiger Umsetzung 2,5 g (8 mMol) 3-Chlorperbenzoesäure nachgegeben. Man setzte nach 4 weiteren Stunden 100 g Wasser zu und neutralisierte die Mischung mit Natriumhydrogenarbonat-Lösung. Die Phasen wurden getrennt, die wäßrige Phase mit Dichlormethan nachextrahiert und das Lösungsmittel abdestilliert. Der Rückstand wurde 4 h mit 100 g 70 %iger Schwefelsäure aufgekocht und danach auf 200 g Eis gegeben. Durch Extraktion und Kristallisation konnten 1,15 g (7,5 mMol, 75 %) 2-Methyl-4-fluorbenzoesäure erhalten werden. Arbeitet man unter diesen Bedingungen statt mit 3-Chlorperbenzoesäure mit 9,2 g (15 mMol) ® Oxone (= Kaliumperoxomonosulfat) so erhält man im wesentlichen dasselbe Ergebnis.

### Beispiel 23

Man tropfte zu einer Lösung von 19,0 (0,1 Mol) 4-Toluolsulfonsäure-Hydrat und 3,8 g (0,1 Mol) 90 % Wasserstoffperoxid in 100 ml Tetrahydrofuran 12,4 g (0,05 Mol) 2-Chlor-4-fluor-4'-methylbenzophenon in 30 ml Tetrahydrofuran. Danach wurde 12 h bei 20 °C weitergerührt und anschließend mit 100 g Wasser verdünnt. Durch weitere Verarbeitung, wie in Beispiel 22 beschrieben, erhielt man 7,6 g (43,5 mMol, 87 %) 2-Chlor-4-fluorbenzoesäure (Fp. 181-186 °C).

### Beispiel 24

7,6 g (25 mMol) 2,4,5-Trichlor-2'-fluorbenzophenon, 4,3 g (0,115 Mol) 90 %iges Wasserstoffperoxid und 0,25 g 2-Nitrobenzoseleninsäure wurden in 50 ml Dichlormethan 24 h gerührt. Danach filtrierte man die unlöslichen Bestandteile ab, versetzte mit Wasser und trennte die organische Phase ab. Nach Entfernung des Lösungsmittels wurde mit 120 g 60 %iger Schwefelsäure 8 h erhitzt, um die vorhandenen Anteile an Ester zu hydrolysieren. Durch anschließende Wasserdampfdestillation konnten 1,55 g (13,8 Mol, 55 %) 2-Fluorphenol abgetrennt werden. Aus der Mutterlauge wurden 4,8 g (21 mMol, 85 %) 2,4,5-Trichlorbenzoesäure (roh (GC) ca. 95 %) isoliert.

### Beispiel 25

Einer Lösung von 2,2 g (10 mMol) 3,4-Difluorbenzophenon und 20 ml Trifluoressigsäure (0 °C) setzte man binnen 1 h 2,5 g Natriumpercarbonat zu und ließ dann auf 20 °C ansteigen. Bei dieser Temperatur wurde 16 h gerührt und dann 50 g Eis zugegeben. Danach extrahierte man zweimal mit Dichlormethan und wusch die organischen Phasen neutral (NaHCO₃). Anschließend wurde das Lösungsmittel entfernt. Man erhielt nach Aufarbeitung, wie in Beispiel 22 beschrieben, 1,2 g (7,6 mMol, 76 %) 3,4-Difluorbenzoesäure, die aus Wasser analysenrein erhalten werden kann (Schmelzpunkt 113-120,5 °C).

### Beispiel 26

In 50 ml 1,2-Dichlorethan wurden 6,0 g (25 mMol) 3,5-Dinitroperbenzoesäure (95 %), 0,1 g 4,4'-Thiobis-(6-tert.butyl-3-methylphenol) und 3,37 g (10 mMol) 2,3,4,5-Tetrachlor-2'(4')-fluorbenzophenon (Isomerengemisch) 8 h zum Rückfluß erhitzt. Danach wurde auf dem Eisbad gekühlt und mit 100 ml 1,2-Dichlorethan verdünnt. Nach vorsichtigem Neutralwaschen der organischen Phase mit Natriumhydrogencarbonat-Lösung wurde das Lösungsmittel entfernt. Der Rückstand wurde, wie in Beispiel 24 beschrieben, weiter aufgearbeitet. Man erhielt 1,2 g 2(4)-Fluorphenol (Rohmischung, Trennung durch Fraktionierung) und 2,1 g (8,1 mMol, 81 %) 2,3,4,5-Tetrachlorbenzoesäure.

## Patentansprüche

1. Verfahren zur Herstellung von halogenierten Benzoesäuren der allgemeinen Formel (1) 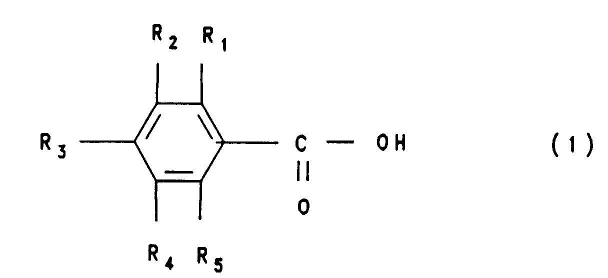 in welcher R₁, R₂, R₃, R₄ und R₅ Wasserstoff-, Fluor-, Chlor- oder Bromatome, Alkyl (C₁-C₆)-, Alkoxy(C₁-C₆)-, Nitro-, Cyano-, Trifluormethyl-, Aldehyd-, Alkoxy(C₁-C₄)-carbonyl-, -SO₂-Alkyl(C₁-C₄)-, -SO₂-Phenyl-, -CONH₂, -CON(Alkyl(C₁-C₄))₂-, Hydroxy-, Carboxyl-, -NH₂- oder -N(Alkyl-(C₁-C₄))₂-gruppen bedeuten, wobei mindestens einer der Substituenten R₁ - R₅ eines der genannten Halogenatome ist, dadurch gekennzeichnet, daß man 1 Mol eines hinsichtlich der Benzolkerne A und B asymmetrisch substituierten Benzophenons der allgemeinen Formel (2) 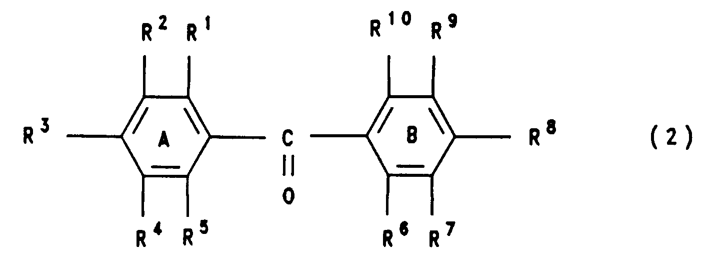 in welcher R¹ - R¹⁰ die vorstehend für R₁ - R₅ genannten Bedeutungen haben, mit 1 bis 10 Mol, vorzugsweise 1,05 bis 3 Mol, eines Oxidationsmittels aus der Reihe Wasserstoffperoxid, Harnstoff-Wasserstoffperoxid-Additionsprodukt, Alkalimetallperoxid, Ammonium-, Alkalimetall- oder Erdalkalimetallperoxodisulfate, -perwolframate, -perborate, -percarbonate, Ozon, Alkyl- oder Arylpercarbonsäuren, Alkyl- oder Arylpersulfonsäuren oder Perschwefelsäure, ggfs. in Gegenwart von gegenüber den Reaktionsteilnehmern inerten anorganischen und/oder organischen Lösungsmitteln bei Temperaturen von -20 ° bis + 100 °C bei Atmosphärendruck, Überdruck oder Unterdruck umsetzt.

2. Verfahren nach Anspruch 1, dadurch gekennzeichnet, daß man bei Temperaturen von 0 °C bis 70 °C umsetzt.

3. Verfahren nach mindestens einem der Ansprüche 1 und 2, dadurch gekennzeichnet, daß man bei Temperaturen von 20 °C bis 60 °C umsetzt.

4. Verfahren nach mindestens einem der Ansprüche 1 bis 3, dadurch gekennzeichnet, daß man mit Natrium-, Kalium- oder Ammoniumperoxodisulfat, Natriumpercarbonat, Perameisensäure, Peressigsäure, Trifluorperessigsäure, Hexafluorpropanpersulfonsäure, 3-Chlorperbenzoesäure, 3,5-Dinitroperbenzoesäure, Magnesiummonoperphthalat, Methanpersulfonsäure, Trifluormethanpersulfonsäure oder p-Toluolpersulfonsäure umsetzt.

5. Verfahren nach mindestens einem der Ansprüche 1 bis 4, dadurch gekennzeichnet, daß man in Gegenwart von Schwefelsäure einer Konzentration von 80 Gewichtsprozent bis 20 gewichtsprozentigem Oleum, Fluorwasserstoff, einer aliphatischen Carbonsäure von 1 bis 6 Kohlenstoffatomen, die im Alkylrest durch Halogenatome substituiert sein kann, einer Alkylsulfonsäure von 1 bis 6 Kohlenstoffatomen, die im Alkylrest durch Halogenatome substituiert sein kann, oder Acetanhydrid umsetzt.

6. Verfahren nach mindestens einem der Ansprüche 1 bis 5, dadurch gekennzeichnet, daß man in Gegenwart von Dichlormethan, Trichlormethan, 1,2-Dichlorethan, Toluol, einem Xylol, Chlorbenzol, Dichlorbenzol, Chlortoluol oder Dichlortoluol umsetzt.

7. Verfahren nach mindestens einem der Ansprüche 1 bis 6, dadurch gekennzeichnet, daß man mit einer 10 bis 90 %igen wäßrigen Wasserstoffperoxidlösung umsetzt.

8. Verfahren nach mindestens einem der Ansprüche 1 bis 7, dadurch gekennzeichnet, daß man mit einer 25 bis 50 %igen wäßrigen Wasserstoffperoxidlösung umsetzt.

9. Verfahren nach mindestens einem der Ansprüche 1 bis 8, dadurch gekennzeichnet, daß man mit einer wäßrigen Wasserstoffperoxidlösung in 80 gewichtsprozentiger Schwefelsäure bis 20 gewichtsprozentigem Oleum umsetzt.

10. Verfahren nach mindestens einem der Ansprüche 1 bis 9, dadurch gekennzeichnet, daß man mit einer wäßrigen Wasserstoffperoxid-Lösung in 95 gewichtsprozentiger Schwefelsäure bis 5 gewichtsprozentigem Oleum umsetzt.

11. Verfahren nach mindestens einem der Ansprüche 1 bis 10, dadurch gekennzeichnet, daß man bei der Umsetzung mit wäßriger Wasserstoffperoxidlösung in Schwefelsäure oder Oleum 200 bis 2000 Massenprozent Schwefelsäure, bezogen auf die Benzophenonausgangsverbindung, einsetzt.

12. Verfahren nach mindestens einem der Ansprüche 1 bis 11, dadurch gekennzeichnet, daß man die als Oxidationsmittel verwendeten Persäuren durch Umsetzung der entsprechenden Säuren mit wäßriger Wasserstoffperoxidlösung, Alkalimetall- oder Erdalkalimetallperoxodisulfaten, -perwolframaten,-perboraten, -percarbonaten, Ozon oder Natriumperoxid in situ herstellt.

13. Verfahren nach mindestens einem der Ansprüche 1 bis 12, dadurch gekennzeichnet, daß man ggfs. intermediär entstehende halogenierte Benzoesäurephenylester in situ oder nach Zwischenisolierung und/oder Reinigung zu den halogenierten Benzoesäuren hydrolysiert.

14. Verfahren nach mindestens einem der Ansprüche 1 bis 13, dadurch gekennzeichnet, daß man die Aufarbeitung des anfallenden Reaktionsgemisches in Gegenwart fluoridfangender Verbindungen durchführt.

15. Verfahren nach mindestens einem der Ansprüche 1 bis 14, dadurch gekennzeichnet, daß man die Aufarbeitung des anfallenden Reaktionsgemisches in Gegenwart von Calciumsalzen oder Siliciumdioxid als fluoridfangenden Verbindungen durchführt.

16. Verfahren nach Anspruch 1, dadurch gekennzeichnet, daß man nach beendeter Umsetzung die Benzoesäuren der Formel (1) durch Verdünnung der Reaktionsmischung und Filtration isoliert und die Phenole der allgemeinen Formel (3) 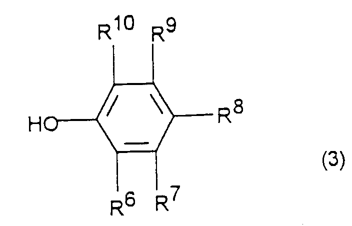 in welcher R₆ - R₁₀ die in Anspruch 1 genannten Bedeutungen haben, wobei mindestens einer der Substituenten R₆ - R₁₀ ein Fluoratom darstellt, nach Verdünnen der Reaktionsmischung und gegebenenfalls Erhitzen zur Desulfonierung durch Wasserdampfextraktion oder Extraktion erhält.

## Claims

1. A process for the preparation of halogenated benzoic acids of the formula (1) 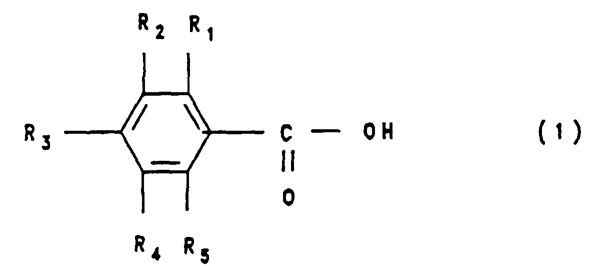 in which R₁, R₂, R₃, R₄ and R₅ are hydrogen, fluorine, chlorine or bromine atoms or C₁-C₆-alkyl, C₁-C₆-alkoxy, nitro, cyano, trifluoromethyl, aldehyde, C₁-C₄-alkoxycarbonyl, -SO₂- (C₁-C₄-alkyl)-, -SO₂-phenyl-, -CONH₂, -CON(C₁-C₄-alkyl)₂, hydroxy, carboxy, -NH₂ or -N(C₁-C₄-alkyl)₂ groups, at least one of the substituents R₁ - R₅ being one of said halogen atoms, which comprises reacting 1 mol of a benzophenone, asymmetrically substituted with respect to the benzene rings A and B, of the formula (2) 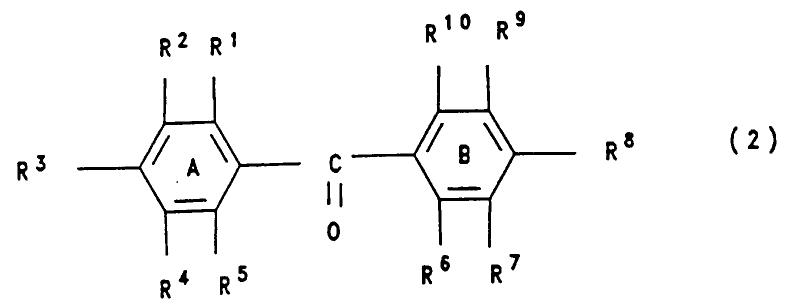 in which R¹ - R¹⁰ are as defined above for R₁ - R₅, with 1 to 10 mol, preferably 1.05 to 3 mol, of an oxidizing agent selected from the group comprising hydrogen peroxide, urea/ hydrogen peroxide addition product, alkali metal peroxide, ammonium, alkali metal or alkaline earth metal peroxodisulfates, pertungstates, perborates or percarbonates, ozone, alkyl- or arylpercarboxylic acids, alkyl- or arylpersulfonic acids or persulfuric acid, optionally in the presence of inorganic and/or organic solvents which are inert towards the reactants, at temperatures from -20° to +100°C, under atmospheric pressure, excess pressure or reduced pressure.

2. The process as claimed in claim 1, wherein the reaction is carried out at temperatures from 0°C to 70°C.

3. The process as claimed in at least one of claims 1 and 2, wherein the reaction is carried out at temperatures from 20°C to 60°C.

4. The process as claimed in at least one of claims 1 to 3, wherein the reaction is carried out with sodium, potassium or ammonium peroxodisulfate, sodium percarbonate, performic acid, peracetic acid, trifluoroperacetic acid, hexafluoropropanepersulfonic acid, 3-chloroperbenzoic acid, 3,5-dinitroperbenzoic acid, magnesium monoperphthalate, methanepersulfonic acid, trifluoromethanepersulfonic acid or p-toluenepersulfonic acid.

5. The process as claimed in at least one of claims 1 to 4, wherein the reaction is carried out in the presence of sulfuric acid having a concentration from 80 percent by weight to 20 percent by weight oleum, hydrogen fluoride, an aliphatic carboxylic acid having 1 to 6 carbon atoms which can be substituted by halogen atoms in the alkyl radical, an alkylsulfonic acid having 1 to 6 carbon atoms which can be substituted by halogen atoms in the alkyl radical, or acetic anhydride.

6. The process as claimed in at least one of claims 1 to 5, wherein the reaction is carried out in the presence of dichloromethane, trichloromethane, 1,2-dichloroethane, toluene, a xylene, chlorobenzene, dichlorobenzene, chlorotoluene or dichlorotoluene.

7. The process as claimed in at least one of claims 1 to 6, wherein the reaction is carried out with a 10 to 90% aqueous hydrogen peroxide solution.

8. The process as claimed in at least one of claims 1 to 7, wherein the reaction is carried out with a 25 to 50% aqueous hydrogen peroxide solution.

9. The process as claimed in at least one of claims 1 to 8, wherein the reaction is carried out with an aqueous hydrogen peroxide solution in 80 percent by weight sulfuric acid to 20 percent by weight oleum.

10. The process as claimed in at least one of claims 1 to 9, wherein the reaction is carried out with an aqueous hydrogen peroxide solution in 95 percent by weight sulfuric acid to 5 percent by weight oleum.

11. The process as claimed in at least one of claims 1 to 10, wherein 200 to 2000 percent by weight of sulfuric acid, based on the benzophenone starting compound, are used in the reaction with aqueous hydrogen peroxide solution in sulfuric acid or oleum.

12. The process as claimed in at least one of claims 1 to 11, wherein the peracids used as oxidizing agents are prepared in situ by reaction of the corresponding acids with aqueous hydrogen peroxide solution, alkali metal or alkaline earth metal peroxodisulfates, pertungstates, perborates or percarbonates, ozone or sodium peroxide.

13. The process as claimed in at least one of claims 1 to 12, wherein any halogenated phenyl benzoates formed as intermediates are hydrolyzed to the halogenated benzoic acids in situ or after intermediate isolation and/or purification.

14. The process as claimed in at least one of claims 1 to 13, wherein the working-up of the reaction mixture obtained is carried out in the presence of fluoride-trapping compounds.

15. The process as claimed in at least one of claims 1 to 14, wherein the working-up of the reaction mixture obtained is carried out in the presence of calcium salts or silicon dioxide as fluoride-trapping compounds.

16. The process as claimed in claim 1, wherein, after the reaction has ended, the benzoic acids of the formula (1) are isolated by dilution of the reaction mixture and filtration and the phenols of the formula (3) 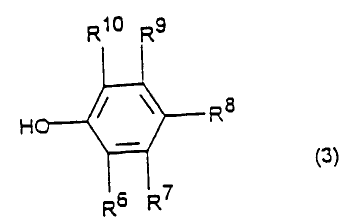 in which R⁶ - R¹⁰ are as defined in claim 1, at least one of the substituents R⁶ - R¹⁰ being a fluorine atom, are obtained, after dilution of the reaction mixture and optionally heating to desulfonate, by steam extraction or extraction.

## Revendications

1. Procédé pour préparer des acides benzoïques halogénés de formule générale (1) : 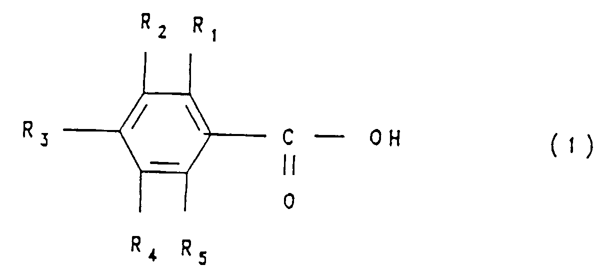 dans laquelle R₁, R₂, R₃, R₄ et R₅ sont des atomes d'hydrogène, de fluor, de chlore ou de brome, ou des groupes alkyle en C₁-C₆, alcoxy en C₁-C₆, nitro, cyano, trifluorométhyle, aldéhyde, (alcoxy en C₁-C₄)-carbonyle, SO₂-alkyle en C₁-C₄, -SO₂-phényle, -CONH₂, -CON(alkyle en C₁-C₄)₂, hydroxy, -carboxyle, -NH₂ ou -N(alkyle en C₁-C₄)₂, où au moins l'un des substituants R₁ à R₅ est l'un des atomes d'halogène mentionnés, caractérisé en ce qu'on fait réagir une mole d'une benzophénone, à substitution asymétrique pour ce qui est des noyaux benzéniques A et B, de formule générale (2) : 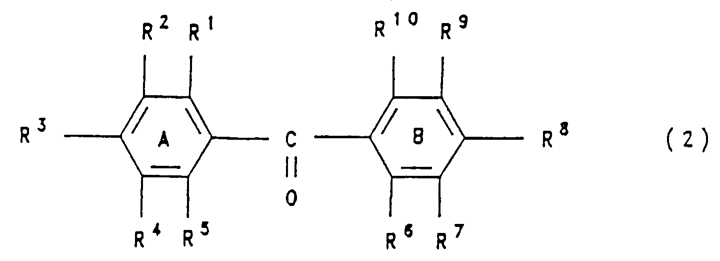 dans laquelle R₁ à R₁₀ ont les significations données ci-dessus pour R₁ à R₅, avec 1 à 10 moles et de préférence de 1,05 à 3 moles d'un oxydant choisi parmi le peroxyde d'hydrogène, les produits d'addition uréeperoxyde d'hydrogène, les peroxydes de métaux alcalins, les peroxodisulfates, pertungstates, perborates, percarbonates d'ammonium, de métaux alcalins ou de métaux alcalino-terreux, l'ozone, les acides alkyl- ou arylpercarboxyliques, les acides alkyl- ou arylpersulfoniques ou l'acide persulfurique, éventuellement en présence de solvants minéraux et/ou organiques inertes vis-à-vis des substances participant à la réaction, à des températures de -20 à +100°C, sous une pression égale, supérieure ou inférieure à la pression atmosphérique.

2. Procédé selon la revendication 1, caractérisé en ce qu'on procède à la réaction à des températures de 0 à 70°C.

3. Procédé selon au moins l'une des revendications 1 et 2, caractérisé en ce qu'on procède à la réaction à des températures de 20 à 60°C.

4. Procédé selon au moins l'une des revendications 1 à 3, caractérisé en ce qu'on procède à la réaction avec du peroxodisulfate de sodium, de potassium ou d'ammonium, du percarbonate de sodium, de l'acide performique, de l'acide peracétique, de l'acide trifluoroperacétique, de l'acide hexafluoropropanepersulfonique, de l'acide 3-chloroperbenzoïque, de l'acide 3,5-dinitroperbenzoïque, du monoperphtalate de magnésium, de l'acide méthanepersulfonique, de l'acide trifluorométhanepersulfonique ou de l'acide paratoluènepersulfonique.

5. Procédé selon au moins l'une des revendications 1 à 4, caractérisé en ce qu'on procède à la réaction en présence d'acide sulfurique à une concentration de 80 à 20 % en poids d'oléum, de fluorure d'hydrogène, d'un acide carboxylique aliphatique ayant de 1 à 6 atomes de carbone et pouvant être substitué dans le fragment alkyle par des atomes d'halogène, d'un acide alkylsulfonique ayant de 1 à 6 atomes de carbone et pouvant être substitué dans le fragment alkyle par des atomes d'halogène, ou d'anhydride acétique.

6. Procédé selon au moins l'une des revendications 1 à 5, caractérisé en ce qu'on procède à la réaction en présence de dichlorométhane, de trichlorométhane, de 1,2-dichloroéthane, de toluène, d'un xylène, de chlorobenzène, de dichlorobenzène, de chlorotoluène ou de dichlorotoluène.

7. Procédé selon au moins l'une des revendications 1 à 6, caractérisé en ce qu'on procède à la réaction avec une solution aqueuse à 10-90 % de peroxyde d'hydrogène.

8. Procédé selon au moins l'une des revendications 1 à 7, caractérisé en ce qu'on procède à la réaction avec une solution aqueuse à 25-50 % de peroxyde d'hydrogène.

9. Procédé selon au moins l'une des revendications 1 à 8, caractérisé en ce qu'on procède à la réaction avec une solution aqueuse de peroxyde d'hydrogène dans de l'acide sulfurique à 80 % en poids jusqu'à de l'oléum à 20 % en poids.

10. Procédé selon au moins l'une des revendications 1 à 9, caractérisé en ce qu'on procède à la réaction avec une solution aqueuse de peroxyde d'hydrogène dans de l'acide sulfurique à 95 % en poids jusqu'à de l'oléum à 5 % en poids.

11. Procédé selon au moins l'une des revendications 1 à 10, caractérisé en ce que, lors de la réaction avec une solution aqueuse de peroxyde d'hydrogène dans de l'acide sulfurique ou de l'oléum, on utilise 200 à 2000 % en masse d'acide sulfurique par rapport au composé de départ benzophénone.

12. Procédé selon au moins l'une des revendications 1 à 11, caractérisé en ce qu'on prépare in situ les peracides utilisés comme oxydants, par réaction des acides correspondants avec une solution aqueuse de peroxyde d'hydrogène, des peroxo disulfates, des pertungstates, des perborates ou des percarbonates de métaux alcalins ou alcalino-terreux, de l'ozone ou du peroxyde de sodium.

13. Procédé selon au moins l'une des revendications 1 à 12, caractérisé en ce qu'on hydrolyse les benzoates de phényle halogénés, éventuellement obtenus en produits intermédiaires, in situ ou après isolement intermédiaire et/ou purification, donnant les acides benzoïques halogénés.

14. Procédé selon au moins l'une des revendications 1 à 13, caractérisé en ce qu'on met en oeuvre le traitement du mélange réactionnel qui se forme, en présence de composés fixateurs de fluorures.

15. Procédé selon au moins l'une des revendications 1 à 14, caractérisé en ce qu'on met en oeuvre le traitement du mélange réactionnel obtenu, en présence de sels de calcium ou de silice en tant que composés fixateurs de fluorure.

16. Procédé selon la revendication 1, caractérisé en ce que, après que la réaction est terminée, on isole les acides benzoïques de formule (1) par dilution du mélange réactionnel et filtration, et, après dilution du mélange réactionnel et éventuellement chauffage pour assurer une désulfonation, on obtient, par extraction à la vapeur d'eau ou extraction, les phénols de formule générale (3) : 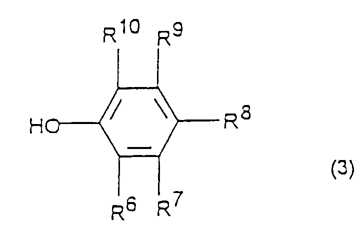 dans laquelle R₆ à R₁₀ ont les significations données dans la revendication 1, où au moins l'un des substituants R₆ à R₁₀ est un atome de fluor.
